# EUROPÄISCHE PATENTSCHRIFT

(11) **EP 2 836 188 B1**
(45) Veröffentlichungstag und Bekanntmachung des Hinweises auf die Patenterteilung: **01.11.2017**
(21) Anmeldenummer: 13700679.7
(22) Anmeldetag: 14.01.2013
(51) Int. Cl.: A61K 8/37, A61K 8/42, A61K 8/55, A61K 8/92, A61Q 19/00

(54) **EMULSION MIT HARNSTOFFDERIVATEN**
EMULSION HAVING UREA DERIVATIVES
ÉMULSION CONTENANT DES DÉRIVÉS D'URÉE

(30) Priorität: 29.02.2012 DE 102012203100
(43) Veröffentlichungstag der Anmeldung: 18.02.2015
(73) Patentinhaber: Henkel AG & Co. KGaA, 40589 Düsseldorf (DE)
(72) Erfinder: BÄHREN, Annika, 41363 Jüchen (DE); WALDMANN-LAUE, Marianne, 40789 Monheim (DE)
(86) Internationale Anmeldenummer: PCT/EP2013/050543
(87) Internationale Veröffentlichungsnummer: WO 2013/127552

(56) Entgegenhaltungen:
- EP-A1- 1 535 607
- EP-A2- 2 181 696
- EP-A2- 2 201 927
- EP-A2- 2 313 076
- DE-A1- 2 703 185
- DE-A1-102006 034 529

## Beschreibung

Die Erfindung betrifft emulsionsförmige Zusammensetzungen, die in Kosmetika, beispielsweise für die Haut oder die Haare, Verwendung finden.

Viele wasserhaltige Zusammensetzungen, insbesondere in Form von Kosmetika, umfassen Öl- oder Fettkomponenten. Diese Öl- oder Fettkomponenten sind nicht wasserlöslich und werden daher z.B. in wasserhaltigen Kosmetika meist mit Hilfe von Emulgatoren in Form einer Emulsion eingearbeitet. Öl- oder Fettkomponenten werden einerseits zur Pflege des Substrats, insbesondere der Haut oder Haare, eingesetzt. Andererseits lassen sich in Ölen oder Fetten öllösliche Wirkstoffe, wie z.B. UV-Filter, in ein wasserhaltiges Kosmetikum in gelöster Form einarbeiten. Die in der Öl- oder Fettkomponente gelösten Wirkstoffe können in dieser Form gleichmäßiger auf das Substrat aufgebracht werden. Auf diese Weise kann sich die Wirkung des Wirkstoffes am Substrat verbessert entfalten.

Kosmetika enthalten oftmals Feuchthaltemittel (im Folgenden Moisturizer genannt), die dem Substrat Feuchtigkeit zuführen, so dass ein trockenes und sprödes Erscheindungsbild des Substrats verhindert oder zumindest eingedämmt wird. Als ein besonders für den Einsatz in Kosmetika geeigneter Moisturizer kennt der Fachmann Verbindungen ausgewählt aus speziellen N-funktionalisierten Harnstoffderivaten mit hydroxyfunktionalisiertem Substituenten (vgl. Formel (I), *vide infra*). Besagte Harnstoffderivate können zwar in eine Emulsion formuliert werden, allerdings verliert die Emulsion an Stabilität. Für die Wiederherstellung der Stabilität der wasserhaltigen Emulsion müssen üblicherweise neben den Emulgatoren zusätzlich verdickend wirkende Polymere als Stabilisatoren eingearbeitet werden.

Aufgabe der vorliegenden Erfindung ist es, wasserhaltige Emulsionen bereitzustellen, die mindestens ein Harnstoff-Derivat der nachfolgenden Formel (I) enthalten und stabil sind. Die Stabilität soll insbesondere in einem Temperaturintervall von 0°C bis 40°C, insbesondere von - 10,0 °C bis + 60°C vorliegen. Die Emulsion soll insbesondere über einen Zeitraum von mehreren Monaten, bevorzugt wenigstens 12 Wochen, stabil bleiben.

Es wurde überraschender Weise gefunden, dass eine Emulsion enthaltend besagte Harnstoff-Derivate und eine spezielle Emulgatorkombination eine hervorragende Stabilität der Emulsion bewirkt, ohne dass zusätzlich auf Stabilisatoren, insbesondere auf verdickend wirkende Polymere zurückgegriffen werden muss. Ferner bleibt die erzielte Viskosität der Emulsion erhalten und ist stabil.

Ein erster Gegenstand der Erfindung ist eine kosmetische Zusammensetzung in Form einer Emulsion, enthaltend
a) mindestens ein Harnstoff-Derivat ausgewählt aus mindestens einer Verbindung gemäß Formel (I), 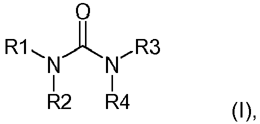 worin die Reste R1, R2, R3 und R4 unabhängig voneinander für ein Wasserstoffatom, eine C₁-C₄-Alkylgruppe, eine C₂-C₆-Alkenylgruppe oder eine C₂-C₆-Hydroxyalkylgruppe mit mindestens einer Hydroxygruppe stehen,
   mit der Maßgabe, dass mindestens einer der besagten Reste eine C₂-C₆Hydroxyalkylgruppe mit mindestens einer Hydroxygruppe bedeutet,
   und
b) mindestens ein Salz von C₁₂₋₂₀-Alkylphosphat, insbesondere ein Salz von Cetylphosphat,
   und
c) mindestens ein C₁₄₋₂₀-Mono- oder Diacylglycerid, bevorzugt mindestens ein C₁₆₋₁₈-Mono- oder Diacylglycerid, besonders bevorzugt ausgewählt aus gehärteten Palmölglyceriden,
   und
d) Wasser,
die C14-20-Mono- oder Diacylglyceride, bevorzugt die C16-18-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung, enthalten sind. Es ist erfindungsgemäß bevorzugt, wenn Wasser in der Emulsion in der kontinuierlichen Phase enthalten ist.

Bevorzugt liegen die erfindungsgemäßen kosmetischen Zusammensetzungen in Form einer flüssigen, fließfähigen oder festen ÖI-in-Wasser-Emulsion, Wasser-in-ÖI-Emulsion, Mehrfach-Emulsion, insbesondere einer Öl-in-Wasser-in-Öl- oder Wasser-in-ÖI-in-Wasser-Emulsion, Makroemulsion, Miniemulsion, Mikroemulsion, PIT-Emulsion, Nanoemulsion, Pickering-Emulsion vor. Erfindungsgemäß besonders bevorzugte kosmetische Zusammensetzungen liegen in Form einer Öl-in-Wasser Emulsion vor.

Weiterhin ist es bevorzugt, wenn die kosmetische Zusammensetzung mindestens 20 Gew.-%, besonders bevorzugt mindestens 40 Gew.-%, ganz besonders bevorzugt mindestens 50 Gew.-% Wasser, insbesondere jeweils in der kontinuierlichen Phase, enthält.

Die Erfindung eignet sich für solche kosmetischen Zusammensetzungen bevorzugt, in denen die Harnstoff-Derivate gemäß Formel (I) in einer Menge von 0,01 bis 10 Gew.-%, vorzugsweise von 0,5 bis 5,0 Gew.-% und insbesondere von 1,2 bis 4,0 Gew.-%, jeweils bezogen auf das Gesamtgewicht der kosmetischen Zusammensetzung, enthalten sind.

Beispiele für erfindungsgemäß in Verbindungen der Formel (I) verwendbare C₁-C₄-Alkylgruppen sind Methyl, Ethyl, n-Propyl, Isopropyl, n-Butyl, sec-Butyl, Isobutyl und tert-Butyl.

Beispiele für erfindungsgemäß in Verbindungen der Formel (I) verwendbare C₂-C₆-Alkenylgruppen sind Vinyl, 2-Propen-1-yl (Allyl) oder 3-Buten-1-yl.

Als bevorzugt geeignete Harnstoffderivate der Formel (I) dienen solche, die mindestens eine C₂-C₆-Hydroxyalkylgruppe mit mindestens einer Hydroxygruppe enthalten, welche aus mindestens einem Vertreter aus der Gruppe aus 2-Hydroxyethyl, 2-Hydroxy-2-methylprop-2-yl, 1,3-Dihydroxy-2-methylprop-2-yl, 1,3-Dihydroxyprop-2-yl, Tris(hydroxymethyl)methyl, 1,3-Dihydroxy-2-hydroxymethylprop-2-yl, 2,3-Dihydroxypropyl, 2-Hydroxypropyl, 3-Hydroxypropyl, 4-Hydroxybutyl, 1-Hydroxy-2-methylprop-2-yl, 2,3,4,5,6-Pentahydroxyhexyl und 1,3,4,5,6-Pentahydroxyhex-2-yl ausgewählt wird. Es ist wiederum bevorzugt, wenn diese bevorzugten Harnstoff-Derivate in einer Menge von 0,01 bis 10 Gew.-%, vorzugsweise von 0,5 bis 5,0 Gew.-% und insbesondere von 2,0 bis 4,0 Gew.-%, jeweils bezogen auf das Gesamtgewicht der kosmetischen Zusammensetzung, enthalten sind.

Es ist erfindungsgemäß bevorzugt, wenn mindestens ein Harnstoff-Derivat ausgewählt wird, bei dem gemäß Formel (I) zwei Reste aus R1, R2, R3 und R4 für eine C₂-C₆-Hydroxyalkylgruppe mit mindestens einer Hydroxygruppe stehen. Es ist wiederum bevorzugt, wenn diese bevorzugten Harnstoff-Derivate gemäß Formel (I) in einer Menge von 0,01 bis 10 Gew.-%, vorzugsweise von 0,5 bis 5,0 Gew.-% und insbesondere von 2,0 bis 4,0 Gew.-%, jeweils bezogen auf das Gesamtgewicht der kosmetischen Zusammensetzung, enthalten sind.

Die bevorzugten C₂-C₆-Hydroxyalkylgruppe mit mindestens einer Hydroxygruppe im Rahmen dieser Ausführungsform, werden aus 2-Hydroxyethyl, 2-Hydroxy-2-methylprop-2-yl, 1,3-Dihydroxy-2-methylprop-2-yl, 1,3-Dihydroxyprop-2-yl, Tris(hydroxymethyl)methyl, 1,3-Dihydroxy-2-hydroxymethylprop-2-yl, 2,3-Dihydroxypropyl, 2-Hydroxypropyl, 3-Hydroxypropyl, 4-Hydroxybutyl, 1-Hydroxy-2-methylprop-2-yl, 2,3,4,5,6-Pentahydroxyhexyl und 1,3,4,5,6-Pentahydroxyhex-2-yl oder deren Gemischen ausgewählt.

Ferner eignen sich solche kosmetischen Zusammensetzungen bevorzugt, die als Harnstoff-Derivat mindestens eine Verbindung gemäß Formel (I) enthalten, wobei einer der Reste R1 oder R2 für eine C₂-C₆-Hydroxyalkylgruppe mit mindestens einer Hydroxygruppe steht und einer der Reste R3 oder R4 für eine C₂-C₆-Hydroxyalkylgruppe mit mindestens einer Hydroxygruppe steht. Es ist wiederum bevorzugt, wenn diese bevorzugten Harnstoff-Derivate gemäß Formel (I) in einer Menge von 0,01 bis 10 Gew.-%, vorzugsweise von 0,5 bis 5,0 Gew.-% und insbesondere von 2,0 bis 4,0 Gew.-%, jeweils bezogen auf das Gesamtgewicht der kosmetischen Zusammensetzung, enthalten sind.

Die bevorzugten C₂-C₆-Hydroxyalkylgruppe mit mindestens einer Hydroxygruppe im Rahmen dieser Ausführungsform, werden unter 2-Hydroxyethyl, 2-Hydroxy-2-methylprop-2-yl, 1,3-Dihydroxy-2-methylprop-2-yl, 1,3-Dihydroxyprop-2-yl, Tris(hydroxymethyl)methyl, 1,3-Dihydroxy-2-hydroxymethylprop-2-yl, 2,3-Dihydroxypropyl, 2-Hydroxypropyl, 3-Hydroxypropyl, 4-Hydroxybutyl, 1-Hydroxy-2-methylprop-2-yl, 2,3,4,5,6-Pentahydroxyhexyl und 1,3,4,5,6-Pentahydroxyhex-2-yl ausgewählt.

Besonders bevorzugte erfindungsgemäße kosmetische Zusammensetzungen enthalten mindestens ein Harnstoff-Derivat, ausgewählt aus einer oder mehrerer Verbindungen der Gruppe, die gebildet wird aus N-(2-Hydroxyethyl)harnstoff, N,N-Bis-(2-hydroxyethyl)harnstoff, N,N'-Bis-(2-hydroxyethyl)harnstoff, N-(3-Hydroxypropyl)harnstoff, N,N-Bis-(3-hydroxypropyl)harnstoff, N,N'-Bis-(3-hydroxypropyl)harnstoff, N-(2-Hydroxypropyl)harnstoff, N,N-Bis-(2-hydroxypropyl)harnstoff, N,N'-Bis-(2-hydroxypropyl)harnstoff, N-(2-Hydroxy-2-methyl-prop-2-yl)harnstoff, N,N-Bis-(2-hydroxy-2-methyl-prop-2-yl)harnstoff, N,N'-Bis-(2-hydroxy-2-methyl-prop-2-yl)harnstoff, N-(1,3-Dihydroxy-2-methyl-prop-2-yl)harnstoff, N,N-Bis-(1,3-dihydroxy-2-methyl-prop-2-yl)harnstoff, N,N'-Bis-(1,3-dihydroxy-2-methyl-prop-2-yl)harnstoff, N-(1,3-Dihydroxy-2-hydroxymethyl-prop-2-yl)harnstoff, N,N-Bis-(1,3-dihydroxy-2-hydroxymethyl-prop-2-yl)harnstoff und N,N'-Bis-(1,3-dihydroxy-2-hydroxymethyl-prop-2-yl)harnstoff. Es ist wiederum bevorzugt, wenn diese besonders bevorzugten Harnstoff-Derivate in einer Menge von 0,01 bis 10 Gew.-%, vorzugsweise von 0,5 bis 5,0 Gew.-% und insbesondere von 2,0 bis 4,0 Gew.-%, jeweils bezogen auf das Gesamtgewicht der kosmetischen Zusammensetzung, enthalten sind.

Ganz besonders bevorzugt wird das Harnstoff-Derivat gemäß Formel (I) ausgewählt aus N,N'-Bis-(2-hydroxyethyl)harnstoff, N-(2-Hydroxyethyl)harnstoff oder einem Gemisch dieser Verbindungen. Besagte ganz besonders bevorzugten Harnstoffderivate sind beispielsweise als Handelsprodukt Hydrovance® von der Firma Akzo Nobel erhältlich. Es ist wiederum bevorzugt, wenn diese ganz besonders bevorzugten Harnstoff-Derivate in einer Menge von 0,01 bis 10 Gew.-%, vorzugsweise von 0,5 bis 5,0 Gew.-% und insbesondere von 2,0 bis 4,0 Gew.-%, jeweils bezogen auf das Gesamtgewicht der kosmetischen Zusammensetzung, enthalten sind.

Neben dem Harnstoffderivat der Formel (I) enthält die erfindungsgemäße kosmetische Zusammensetzung zusätzlich zwingend mindestens ein Salz von C₁₂₋₂₀-Alkylphosphaten, insbesondere mindestens ein Salz des Cetylphosphats.

Salze von C₁₂₋₂₀-Alkylphosphaten, umfassen Phosphorsäure-C₁₂₋₂₀-Monoalkylester und Phosphorsäure-C₁₂₋₂₀-Dialkylester.

Die Salze von C₁₂₋₂₀-Alkylphosphaten können beispielsweise als Alkali-, Erdalkali-, Ammonium-, Alkylammonium-, Alkanolamin- oder Glucammoniumsalz vorliegen. Bevorzugt sind die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze, sowie Salze der basischen Aminosäuren Ornithin, Lysin, Arginin und/oder Histidin. Besonders bevorzugt sind die Kaliumsalze besagter C₁₂₋₂₀-Alkylphosphate.

Erfindungsgemäß bevorzugte kosmetische Zusammensetzungen sind dadurch gekennzeichnet, dass die Salze von C₁₂₋₂₀-Alkylphosphat, insbesondere die Salze von Cetylphosphat" in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten sind.

Es sind solche kosmetischen Zusammensetzungen bevorzugt, die sich dadurch kennzeichnen, dass das Gewichtverhältnis von den besagten Harnstoff-Derivaten zu den Salzen von C₁₂₋₂₀-Alkylphosphat (insbesondere zu den Salzen von Cetylphosphat) in einem Bereich von 1 zu 0,04 bis 1 zu 4, bevorzugt von 1 zu 0,05 bis 1 zu 1, besonders bevorzugt von 1 bis 0,075 zu 1 bis 0,35, liegt.

Im Rahmen einer besonders bevorzugten Ausführungsform der Erfindung enthält die erfindungsgemäße kosmetische Zusammensetzung die Salze von C₁₂₋₂₀-Alkylphosphat, insbesondere die Salze von Cetylphosphat
- in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten
   und
- in einem Gewichtverhältnis von den besagten Harnstoff-Derivaten zu den Salzen von C₁₂₋₂₀-Alkylphosphat (insbesondere zu den Salzen von Cetylphosphat) in einem Bereich von 1 zu 0,04 bis 1 zu 4, bevorzugt von 1 zu 0,05 bis 1 zu 1, besonders bevorzugt von 1 bis 0,075 zu 1 bis 0,35.

Erfindungsgemäß bevorzugte Salze von C₁₂₋₂₀-Alkylphosphaten, sind ausgewählt aus den Monoestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäuremonoester. Besonders bevorzugt ist Dikaliummonocetylphosphat.

Weitere erfindungsgemäß bevorzugte Salze von C₁₂₋₂₀-Alkylphosphaten sind ausgewählt aus den Diestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäurediester. Besonders bevorzugt ist Kaliumdicetylphosphat.

Besonders bevorzugt sind Mischungen aus Mono-C₁₂₋₂₀-Alkylphosphaten und Di-C₁₂₋₂₀-Alkylphosphaten. Außerordentlich bevorzugt sind Mischungen aus Dikaliummonocetylphosphat und Kaliumdicetylphosphat.

Die erfindungsgemäße kosmetische Zusammensetzung enthält zwingend mindestens ein C₁₄₋₂₀-Mono- oder Diacylglycerid, bevorzugt mindestens ein C₁₆₋₁₈-Mono- oder Diacylglycerid. Diese werden jeweils besonders bevorzugt aus gehärteten Palmölglyceriden ausgewählt. Sie tragen die INCI-Deklaration Hydrogenated Palm Glycerides.

Erfindungsgemäß bevorzugte C₁₄₋₂₀-Mono- oder Diacylglyceride sind ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid, Diarachinoylglycerid sowie Mischungen zweier oder meherer Verbindungen daraus. Weitere erfindungsgemäß bevorzugte C₁₄₋₂₀-Mono- oder Diacylglyceride sind Glyceride gehärteter, das heißt, hydrierter, bevorzugt vollständig hydrierter, Fettsäuren natürlicher Öle. Erfindungsgemäß besonders bevorzugt sind gehärtete Palmölglyceride.

Erfindungsgemäß kosmetische Zusammensetzungen enthalten die C₁₄₋₂₀-Mono- oder Diacylglyceride, bevorzugt die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung.

Der erfindungsgemäße Effekt wird besonders ausgeprägt, wenn die Salze von C₁₂₋₂₀-Alkylphosphat (insbesondere die Salze von Cetylphosphat) und die C₁₄₋₂₀-Mono- und C₁₄₋₂₀-Diacylglyceride bevorzugt in einem Gewichtsverhältnis C₁₂₋₂₀-Alkylphosphat-Salz zu besagtem Glycerid von 3 zu 1 bis 1 zu 1,5, besonders bevorzugt von 2,5 zu 1 bis 1 zu 1, in der erfindungsgemäßen Zusammensetzung enthalten sind.

Es ist erfindungsgemäß besonders bevorzugt, wenn die erfindungsgemäße kosmetische Zusammensetzung
- die Salze von C₁₂₋₂₀-Alkylphosphat (insbesondere die Salze von Cetylphosphat) und die C₁₄₋₂₀-Mono- und C₁₄₋₂₀-Diacylglyceride in einem Bereich des Gewichtsverhältnisses C₁₂₋₂₀-Alkylphosphat-Salz zu besagtem Glycerid von 3 zu 1 bis 1 zu 1,5, bevorzugt von 2,5 zu 1 bis 1 zu 1, und
- die Salze von C₁₂₋₂₀-Alkylphosphat, insbesondere die Salze von Cetylphosphat in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten, und
- die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung,
enthält. Dabei ist es wiederum bevorzugt, den erfindungsgemäßen Zusammensetzungen dieser Ausführungsform die Harnstoff-Derivate der Formel (I) bzw. deren bevorzugte Vertreter in den bevorzugten Mengen (vide supra) zuzusetzen. Es ist erfindungsgemäß ganz besonders bevorzugt, wenn die erfindungsgemäße kosmetische Zusammensetzung
- die Harnstoff-Derivate gemäß Formel (I), insbesondere deren bevorzugten Vertreter, in einer Menge von 0,01 bis 10 Gew.-%, vorzugsweise von 0,5 bis 5,0 Gew.-% und insbesondere von 1,2 bis 4,0 Gew.-%, jeweils bezogen auf das Gesamtgewicht der kosmetischen Zusammensetzung, und
- die Salze von C₁₂₋₂₀-Alkylphosphat, insbesondere die Salze von Cetylphosphat in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten, und
- die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung, und
- die Salze von C₁₂₋₂₀-Alkylphosphat (insbesondere die Salze von Cetylphosphat) und die C_{14>-20}-Mono- und C₁₄₋₂₀-Diacylglyceride in einem Bereich des Gewichtsverhältnisses C₁₂₋₂₀-Alkylphosphat-Salz zu besagtem Glycerid von 3 zu 1 bis 1 zu 1,5, bevorzugt von 2,5 zu 1 bis 1 zu 1, und
- die besagten Harnstoff-Derivate und die Salze von C₁₂₋₂₀-Alkylphosphat (insbesondere den den Salzen von Cetylphosphat) in einem Bereich des Gewichtsverhältnisses C₁₂₋₂₀-Alkylphosphat-Salz zu besagtem Glycerid von 1 zu 0,04 bis 1 zu 4, bevorzugt von 1 zu 0,05 bis 1 zu 1, besonders bevorzugt von 1 bis 0,075 zu 1 bis 0,35
enthält.

Optionale Zusatzstoffe sind verdickend wirkende Polymere, z. B. anionische Polymere aus Acrylsäure, Methacrylsäure, Crotonsäure, Maleinsäureanhydrid und 2-Acrylamido-2-methylpropan-sulfonsäure, wobei die sauren Gruppen ganz oder teilweise als Natrium-, Kalium-, Ammonium-, Mono- oder Triethanolamin-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel® 305, Simulgel® 600, Simulgel® NS, Simulgel® EPG und Simulgel® EG von SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol®. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen® und die Carbopol®-Typen 954, 980, 1342 und ETD 2020 (ex Noveon/Lubrizol).

Es ist jedoch erfindungsgemäß bevorzugt, verdickend wirkende Polymere gar nicht oder in geringen Mengen zuzusetzen. Aus diesem Grunde sind solche kosmetischen Zusammensetzungen bevorzugt, die sich dadurch kennzeichnen, dass verdickend wirkende Polymere in einer Menge von 0 bis 0,5 Gew.-%, besonders bevorzugt von 0 bis 0,4 Gew.-%, ganz besonders bevorzugt von 0 bis 0,1 Gew., am bevorzugtesten von 0 bis 0,05 Gew.-%, jeweils bezogen auf das Gesamtgewicht der Zusammensetzung, enthalten sind.

Weitere erfindungsgemäß bevorzugte Zusammensetzungen sind dadurch gekennzeichnet, dass sie mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.

Die erfindungsgemäßen Zusammensetzungen enthalten bevorzugt mindestens einen Fettkörper. Als Fettkörper besonders bevorzugt mindestens einen Fettstoff. Unter sind Fettsäuren, Fettalkohole, natürliche und synthetische Wachse und natürliche und synthetische kosmetische Ölkomponenten zu verstehen.

Die Fettstoffe sind in den erfindungsgemäßen Zusammensetzungen bevorzugt in einer Menge von 5,0 bis 50,0 Gew.-%, besonders bevorzugt von 8,0 bis 30,0 Gew.-%, jeweils bezogen auf das Gesamtgewicht des Zusammensetzung, enthalten.

Als Fettstoff eignen sich bevorzugt lineare, gesättigte primäre Alkohole mit 14 - 30 Kohlenstoffatomen, Fettsäuren mit 6 bis 30 Kohlenstoffatomen, Triglyzeride, Wachse, Fettsäureester, natürliche Öle, Silikone.

Als eine Komponente des Fettkörpers enthalten die erfindungsgemäßen Zusammensetzungen bevorzugt mindestens einen linearen, gesättigten primären Alkohol mit 14 - 30 Kohlenstoffatomen.

Erfindungsgemäß bevorzugte lineare, gesättigte primäre Alkohole mit 14 - 30 Kohlenstoffatomen sind ausgewählt aus Myristylalkohol, Cetylalkohol, Stearylalkohol, 12-Hydroxystearylalkohol, Arachidylalkohol (Arachylalkohol), Behenylalkohol, Lignocerylalkohol, Cerylalkohol und Myricylalkohol und Mischungen hiervon, insbesondere technische Mischungen wie Arachidylalkohol und Behenylalkohol sowie Cetylalkohol und Stearylalkohol (Cetearylalkohol). Besonders bevorzugt sind Cetylalkohol, Stearylalkohol, Arachidylalkohol und Behenylalkohol und Mischungen hiervon. Besonders bevorzugt sind Mischungen aus Cetearylalkohol und Behenylalkohol.

Die vorgenannten Alkohole werden im Folgenden auch synonym als "Fettalkohole" bezeichnet.

Bevorzugte kosmetische Zusammensetzungen enthalten mindestens einen linearen, gesättigten primären Alkohol mit 14 - 30 Kohlenstoffatomen in einer Gesamtmenge von 0,5 bis 10,0 Gew.-%, bevorzugt 1,0 bis 8,0 Gew.-%, jeweils bezogen auf das Gesamtgewicht der Zusammensetzung.

Als weitere Fettstoffe können die erfindungsgemäßen Zusammensetzungen bevorzugt mindestens eine Fettsäure mit 6 bis 30 Kohlenstoffatomen enthalten.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge an Fettsäure beträgt dabei bevorzugt 0,1 bis 15 Gew.%, bezogen auf das gesamte Zusammensetzung. In einer besonders bevorzugten Ausführungsform beträgt die Menge 0,5 bis 10 Gew.%, wobei ganz besonders vorteilhaft Mengen von 1 bis 5 Gew.% sind.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, eignen sich bevorzugt:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle, insbesondere Capryl-/Caprinsäuretriglyzerid.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Triglyceriden gesättigter und gegebenenfalls hydroxylierter C₁₆₋₃₀-Fettsäuren, z. B. gehärteten Triglyceridfetten, insbesondere Triglyceride aus gehärtetem Palmöl.
- Phospholipiden, beispielsweise Sojalecithin, Ei-Lecithin und Kephalinen,
- Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/ oder Hydroxy-Gruppen enthalten, bevorzugt Substanzen mit den INCI-Bezeichnungen Dimethiconol, Amodimethicone oder Trimethylsilylamodimethicone.

Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäße Zusammensetzung.

Weitere mögliche Inhaltsstoffe der erfindungsgemäßen kosmetischen Zusammensetzungen können über ihre Funktion definiert werden. Natürlich können manche Inhaltsstoffe auch multifunktionell sein. Bevorzugte Inhaltsstoffe der erfindungsgemäßen kosmetischen Zusammensetzungen können sein: Absorptionsmittel, antimikrobielle Stoffe, Bindemittel, Bleichmittel, Chelatbildner, Emollientien, Enthaarungsmittel und Wirkstoffe mit Haarwuchs inhibierender Wirkung, Feuchtigkeitsspender, Filmbildner, Farbstoffe, Konservierungsstoffe, Korrosionsschutzmittel, hautkühlende Wirkstoffe, pH-Wert-Regler/ Puffersubstanzen, Treibgase, Trübungsmittel, UV-Absorber/ Lichtfiltersubstanzen, Vergällungsmittel. Ferner können auch Kombinationen der vorgenannten Inhaltsstoffe in den erfindungsgemäß bevorzugten Zusammensetzungen enthalten sein.

### Beispiele

Es wurde folgende ÖI-in-Wasser-Emulsion als Hautpflegemittel nach einem Standard Emulgierverfahren unter Einsatz der erfindungsgemäßen Inhaltsstoffe hergestellt:

| **Inhaltsstoffe (INCI-Bezeichnung)** | **Gew.-%** |
|---|---|
| Hydroxyethyl Urea | 1,50 |
| Potassium Cetyl Phosphate | 0,63 |
| Hydrogenated Palm Glycerides | 0,37 |
| Glyceryl Stearate | 0,50 |
| Glycerin | 5,00 |
| Sorbitol | 2,10 |
| Ethylhexyl Palmitate | 2,00 |
| Cetearyl Alcohol | 1,00 |
| Urea | 0,10 |
| Panthenol | 0,10 |
| Tocopheryl Acetate | 0,05 |
| Sodium Carbomer | 0,30 |
| Phenoxyethanol | 0,70 |
| Ethylparaben | 0,10 |
| Methylparaben | 0,10 |
| Parfum (Fragrance) | 0,15 |
| Aqua | ad 100 |

Als Quelle für Hydroxyethyl Urea wurde der Rohstoff Hydrovance® der Firma Akzo Nobel eingesetzt.

Obige Emulsion war bei -10°C und 60°C für einen Zeitraum von 12 Wochen lagerstabil und wies ein stabiles Viskositätsprofil auf.

## Patentansprüche

1. Kosmetische Zusammensetzung in Form einer Emulsion, enthaltend
• mindestens ein Harnstoff-Derivat ausgewählt aus mindestens einer Verbindung gemäß Formel (I), 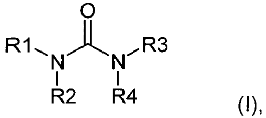 worin die Reste R1, R2, R3 und R4 unabhängig voneinander für ein Wasserstoffatom, eine C₁-C₄-Alkylgruppe, eine C₂₋C₆-Alkenylgruppe oder eine C₂-C₆-Hydroxyalkylgruppe mit mindestens einer Hydroxygruppe stehen,
mit der Maßgabe, dass mindestens einer der besagten Reste eine C₂-C₆Hydroxyalkylgruppe mit mindestens einer Hydroxygruppe bedeutet,
und
• mindestens ein Salz von C₁₂₋₂₀-Alkylphosphat, insbesondere ein Salz von Cetylphosphat, und
• mindestens ein C₁₄₋₂₀-Mono- oder Diacylglycerid, bevorzugt mindestens ein C₁₆₋₁₈-Mono- oder Diacylglycerid, besonders bevorzugt ausgewählt aus gehärteten Palmölglyceriden,
und
• Wasser, wobei
die C₁₄₋₂₀-Mono- oder Diacylglyceride, bevorzugt die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung, enthalten sind.

2. Kosmetische Zusammensetzung gemäß Anspruch 1 **dadurch gekennzeichnet, dass** gemäß Formel (I) zwei Reste aus R1, R2, R3 und R4 für eine C₂-C₆-Hydroxyalkylgruppe mit mindestens einer Hydroxygruppe stehen.

3. Kosmetische Zusammensetzung gemäß Anspruch 1 oder 2, **dadurch gekennzeichnet, dass** gemäß Formel (I) einer der Reste R1 oder R2 für eine C₂-C₆-Hydroxyalkylgruppe mit mindestens einer Hydroxygruppe steht und einer der Reste R3 oder R4 für eine C₂-C₆-Hydroxyalkylgruppe mit mindestens einer Hydroxygruppe steht.

4. Kosmetische Zusammensetzung gemäß einem der Ansprüche 1 bis 3, **dadurch gekennzeichnet, dass** sie mindestens ein Harnstoff-Derivat enthält, ausgewählt aus einer oder mehrerer Verbindungen der Gruppe, die gebildet wird aus N-(2-Hydroxyethyl)harnstoff, N,N-Bis-(2-hydroxyethyl)harnstoff, N,N'-Bis-(2-hydroxyethyl)harnstoff, N-(3-Hydroxypropyl)harnstoff, N,N-Bis-(3-hydroxypropyl)harnstoff, N,N'-Bis-(3-hydroxypropyl)harnstoff, N-(2-Hydroxypropyl)harnstoff, N,N-Bis-(2-hydroxypropyl)harnstoff, N,N'-Bis-(2-hydroxypropyl)-harnstoff, N-(2-Hydroxy-2-methyl-prop-2-yl)harnstoff, N,N-Bis-(2-hydroxy-2-methyl-prop-2-yl)harnstoff, N,N'-Bis-(2-hydroxy-2-methyl-prop-2-yl)harnstoff, N-(1,3-Dihydroxy-2-methyl-prop-2-yl)harnstoff, N,N-Bis-(1,3-dihydroxy-2-methyl-prop-2-yl)harnstoff, N,N'-Bis-(1,3-dihydroxy-2-methyl-prop-2-yl)harnstoff, N-(1,3-Dihydroxy-2-hydroxymethyl-prop-2-yl)harnstoff, N,N-Bis-(1,3-dihydroxy-2-hydroxymethyl-prop-2-yl)harnstoff und N,N'-Bis-(1,3-dihydroxy-2-hydroxymethyl-prop-2-yl)harnstoff.

5. Kosmetische Zusammensetzung gemäß einem der Ansprüche 1 bis 4, **dadurch gekennzeichnet, dass** das Harnstoff-Derivat ausgewählt wird aus N,N'-Bis-(2-hydroxyethyl)harnstoff, N-(2-Hydroxyethyl)harnstoff oder einem Gemisch dieser Verbindungen.

6. Kosmetische Zusammensetzung gemäß einem der Ansprüche 1 bis 5 **dadurch gekennzeichnet, dass** besagte Harnstoff-Derivate in einer Menge von 0,01 bis 10 Gew.-%, vorzugsweise von 0,5 bis 5,0 Gew.-% und insbesondere von 2,0 bis 4,0 Gew.-%, jeweils bezogen auf das Gesamtgewicht der kosmetischen Zusammensetzung, enthalten sind.

7. Kosmetische Zusammensetzung nach einem der Ansprüche 1 bis 6, **dadurch gekennzeichnet, dass** die Salze von C₁₂₋₂₀-Alkylphosphat, insbesondere die Salze von Cetylphosphat" in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten sind.

8. Kosmetische Zusammensetzung gemäß einem der Ansprüche 1 bis 7, **dadurch gekennzeichnet, dass** das Gewichtverhältnis von den besagten Harnstoff-Derivaten zu den Salzen von C₁₂₋₂₀-Alkylphosphat (insbesondere zu den Salzen von Cetylphosphat) in einem Bereich von 1 zu 0,04 bis 1 zu 4, bevorzugt von 1 zu 0,05 bis 1 zu 1, besonders bevorzugt von 1 bis 0,075 zu 1 bis 0,35, liegt.

9. Kosmetische Zusammensetzung gemäß einem der Ansprüche 1 bis 8, **dadurch gekennzeichnet, dass** die Salze von C₁₂₋₂₀-Alkylphosphat (insbesondere die Salze von Cetylphosphat) und die C₁₄₋₂₀-Mono- und C₁₄₋₂₀-Diacylglyceride in einem Gewichtsverhältnis C₁₂₋₂₀-Alkylphosphat-Salz zu besagtem Glycerid von 3 zu 1 bis 1 zu 1,5, bevorzugt von 2,5 zu 1 bis 1 zu 1, in der enthalten sind.

10. Kosmetische Zusammensetzung gemäß einem der Ansprüche 1 bis 9, **dadurch gekennzeichnet, dass** verdickend wirkende Polymere in einer Menge von 0 bis 0,5 Gew.-%, besonders bevorzugt von 0 bis 0,4 Gew.-%, ganz besonders bevorzugt von 0 bis 0,1 Gew., am bevorzugtesten von 0 bis 0,05 Gew.-%, jeweils bezogen auf das Gesamtgewicht der Zusammensetzung, enthalten sind.

11. Kosmetische Zusammensetzung gemäß einem der Ansprüche 1 bis 10, **dadurch gekennzeichnet, dass** es als ÖI-in-Wasser-Emulsion vorliegt.

12. Kosmetische Zusammensetzung gemäß einem der Ansprüche 1 bis 11, **dadurch gekennzeichnet, dass** Wasser in einer Menge von umfasst.

13. Kosmetische Zusammensetzung gemäß einem der Ansprüche 1 bis 12, **dadurch gekennzeichnet, dass** sie Wasser in der kontinuierlichen Phase enthält.

14. Kosmetische Zusammensetzung gemäß einem der Ansprüche 1 bis 13, **dadurch gekennzeichnet, dass** sie mindestens 20 Gew.-%, bevorzugt mindestens 40 Gew.-%, besonders bevorzugt mindestens 50 Gew.-% Wasser, insbesondere in der kontinuierlichen Phase, enthält.

## Claims

1. A cosmetic composition in the form of an emulsion, containing
• at least one urea derivative selected from at least one compound according to formula (I), 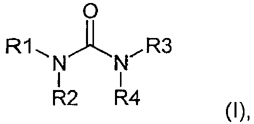 in which the functional groups R1, R2, R3 and R4 represent, independently of one another, a hydrogen atom, a C₁-C₄ alkyl group, a C₂-C₆ alkenyl group or a C₂-C₆ hydroxyalkyl group comprising at least one hydroxy group, with the proviso that at least one of said functional groups represents a C₂-C₆ hydroxyalkyl group comprising at least one hydroxy group, and
• at least one salt of C₁₂₋₂₀ alkyl phosphate, in particular a salt of cetyl phosphate, and
• at least one C₁₄₋₂₀ monoacylglyceride or diacylglyceride, preferably at least one C₁₆₋₁₈ monoacylglyceride or diacylglyceride, particularly preferably selected from hydrogenated palm glycerides, and
• water,
wherein the C₁₄₋₂₀ monoacylglycerides or diacylglycerides, preferably the C₁₆₋₁₈ monoacylglycerides or diacylglycerides, particularly preferably the hydrogenated palm glycerides, are contained in a total amount of from 0.1 to 1.7 wt.%, based on the total weight of the composition.

2. The cosmetic composition according to claim 1, **characterized in that**, according to formula (I), two functional groups from R1, R2, R3 and R4 represent a C₂-C₆ hydroxyalkyl group comprising at least one hydroxy group.

3. The cosmetic composition according to claim 1 or 2, **characterized in that**, according to formula (I), one of the functional groups R1 and R2 represents a C₂-C₆ hydroxyalkyl group comprising at least one hydroxy group, and one of the functional groups R3 and R4 represents a C₂-C₆ hydroxyalkyl group comprising at least one hydroxy group.

4. The cosmetic composition according to one of claims 1 to 3, **characterized in that** it contains at least one urea derivative, selected from one or more compounds of the group formed of N-(2-hydroxyethyl)urea, N,N-bis-(2-hydroxyethyl)urea, N,N'-bis-(2-hydroxyethyl)urea, N-(3-hydroxypropyl)urea, N,N-bis-(3-hydroxypropyl)urea, N,N'-bis-(3-hydroxypropyl)urea, N-(2-hydroxypropyl)urea, N,N-bis-(2-hydroxypropyl)urea, N, N'-bis-(2-hydroxypropyl)-urea, N-(2-hydroxy-2-methyl-prop-2-yl)urea, N,N-bis-(2-hydroxy-2-methyl-prop-2-yl)urea, N,N'-bis-(2-hydroxy-2-methyl-prop-2-yl)urea, N-(1,3-dihydroxy-2-methyl-prop-2-yl)urea, N,N-bis-(1,3-dihydroxy-2-methyl-prop-2-yl)urea, N,N'-bis-(1,3-dihydroxy-2-methyl-prop-2-yl)urea, N-(1,3-dihydroxy-2-hydroxymethyl-prop-2-yl)urea, N,N-bis-(1,3-dihydroxy-2-hydroxymethyl-prop-2-yl)urea and N,N'-bis-(1,3-dihydroxy-2-hydroxymethyl-prop-2-yl)urea.

5. The cosmetic composition according to one of claims 1 to 4, **characterized in that** the urea derivative is selected from N,N'-bis-(2-hydroxyethyl)urea, N-(2-hydroxyethyl)urea or a mixture of these compounds.

6. The cosmetic composition according to one of claims 1 to 5, **characterized in that** said urea derivatives are contained in an amount of from 0.01 to 10 wt.%, preferably of from 0.5 to 5.0 wt.% and in particular of from 2.0 to 4.0 wt.%, in each case based on the total weight of the cosmetic composition.

7. The cosmetic composition according to one of claims 1 to 6, **characterized in that** the salts of C₁₂₋₂₀ alkyl phosphate, in particular the salts of cetyl phosphate, are contained in a total amount of from 0.2 to 2.0 wt.%, preferably of from 0.3 to 1.8 wt.%, particularly preferably of from 0.3 to 0.7 wt.%, in each case based on the total weight of the composition.

8. The cosmetic composition according to one of claims 1 to 7, **characterized in that** the weight ratio of said urea derivatives to the salts of C₁₂₋₂₀ alkyl phosphate (in particular to the salts of cetyl phosphate) is in a range of from 1:0.04 to 1:4, preferably of from 1:0.05 to 1:1, particularly preferably of from 1-0.075 to 1-0.35.

9. The cosmetic composition according to one of claims 1 to 8, **characterized in that** the salts of C₁₂₋₂₀ alkyl phosphate (in particular the salts of cetyl phosphate) and the C₁₄₋₂₀ monoacylglycerides and C₁₄₋₂₀ diacylglycerides are contained therein in a weight ratio of C₁₂₋₂₀ alkyl phosphate salt to said glyceride of from 3:1 to 1:1.5, preferably of from 2.5:1 to 1:1.

10. The cosmetic composition according to one of claims 1 to 9, **characterized in that** thickening polymers are contained in an amount of from 0 to 0.5 wt.%, particularly preferably of from 0 to 0.4 wt.%, more particularly preferably of from 0 to 0.1 wt.%, most preferably of from 0 to 0.05 wt.%, in each case based on the total weight of the composition.

11. The cosmetic composition according to one of claims 1 to 10, **characterized in that** it is in the form of an oil-in-water emulsion.

12. The cosmetic composition according to one of claims 1 to 11, **characterized in that** it comprises water in an amount of.

13. The cosmetic composition according to one of claims 1 to 12, **characterized in that** it contains water in the continuous phase.

14. The cosmetic composition according to one of claims 1 to 13, **characterized in that** it contains at least 20 wt.%, preferably at least 40 wt.%, particularly preferably at least 50 wt.%, of water, in particular in the continuous phase.

## Revendications

1. Composition cosmétique se présentant sous la forme d'une émulsion contenant
• au moins un dérivé de l'urée choisi parmi au moins un composé de la formule (I), 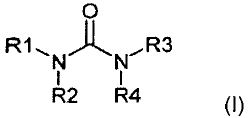 dans laquelle les radicaux R1, R2, R3 et R4 représentent, indépendamment les uns des autres, un atome d'hydrogène, un groupe alkyle en C₁ à C₄, un groupe alcényle en C₂ à C₆ ou un groupe hydroxyalkyle en C₂ à C₆ comportant au moins un groupe hydroxyle, dans la mesure où au moins un desdits radicaux est un groupe hydroxyalkyle en C₂ à C₆ comportant au moins un groupe hydroxyle, et
• au moins un sel de phosphate d'alkyle en C₁₂ à C₂₀, notamment un sel de phosphate de cétyle, et
• au moins un mono- ou diacylglycéride en C₁₄ à C₂₀, de préférence au moins un mono- ou diacylglycéride en C₁₆ à C₁₈, de manière particulièrement préférée choisis parmi les glycérides d'huile de palme durcis, et
• de l'eau,
les mono- ou diacylglycérides en C₁₄ à C₂₀, de préférence les mono- ou diacylglycérides en C₁₆ à C₁₈, de manière particulièrement préférée les glycérides d'huile de palme durcis étant présents dans une quantité totale de 0,1 à 1,7% en poids par rapport au poids total de la composition.

2. Composition cosmétique selon la revendication 1, **caractérisée en ce que** deux radicaux parmi R1, R2, R3 et R4 de la formule (I) représentent un groupe hydroxyalkyle en C₂ à C₆ comportant au moins un groupe hydroxyle.

3. Composition cosmétique selon la revendication 1 ou 2, **caractérisée en ce que** l'un des radicaux R1 ou R2 de la formule (I) représente un groupe hydroxyalkyle en C₂ à C₆ comportant au moins un groupe hydroxyle de la formule (I) et l'un des radicaux R3 ou R4 représente un groupe hydroxyalkyle en C₂ à C₆ comportant au moins un groupe hydroxyle.

4. Composition cosmétique selon l'une des revendications 1 à 3, **caractérisée en ce qu'**elle contient au moins un dérivé de l'urée choisi parmi un ou plusieurs composés du groupe formé la N-(2-hydroxyéthyl)urée, la N,N-Bis-(2-hydroxyéthyl)urée, la N,N'-Bis-(2-hydroxyéthyl)urée, la N-(3-hydroxypropyl)urée, la N,N-Bis-(3-hydroxypropyl)urée, la N,N'-Bis-(3-hydroxypropyl)urée, la N-(2-hydroxypropyl)urée, la N,N-Bis-(2-hydroxypropyl)urée, la N,N'-Bis-(2-hydroxypropyl)-urée, la N-(2-hydroxy-2-méthyl-prop-2-yl)urée, la N,N-Bis-(2-hydroxy-2-méthyl-prop-2-yl)urée, la N,N'-Bis-(2-hydroxy-2-méthyl-prop-2-yl)urée, la N-(1,3-dihydroxy-2-méthyl-prop-2-yl)urée, la N,N-Bis-(1,3-dihydroxy-2-méthyl-prop-2-yl)urée, la N,N'-Bis-(1,3-dihydroxy-2-méthyl-prop-2-yl)urée, la N-(1,3-dihydroxy-2-hydroxyméthyl-prop-2-yl)urée, la N,N-Bis-(1,3-dihydroxy-2-hydroxyméthyl-prop-2-yl)urée et la N,N'-Bis-(1,3-dihydroxy-2-hydroxyméthyl-prop-2-yl)urée.

5. Composition cosmétique selon l'une des revendications 1 à 4, **caractérisée en ce que** le dérivé de l'urée est choisi parmi la N,N'-bis-(2-hydroxyéthyl)urée, la N-(2-hydroxyéthyl)urée ou un mélange de ces composés.

6. Composition cosmétique selon la revendication 1 à 5, **caractérisée en ce que** les dérivés de l'urée sont présents dans une quantité de 0,01 à 10% en poids, de préférence de 0,5 à 5,0% en poids et en particulier de 2,0 à 4,0% en poids, par rapport au poids total de la composition cosmétique.

7. Composition cosmétique selon l'une des revendications 1 à 6, **caractérisée en ce que** les sels de phosphate d'alkyle en C₁₂ à C₂₀, en particulier les sels de phosphate de cétyle, sont présents dans une quantité totale de 0,2 à 2,0% en poids, de préférence de 0,3 % à 1,8% en poids, de manière particulièrement préférée de 0,3 à 0,7% en poids, par rapport au poids total de la composition.

8. Composition cosmétique selon l'une des revendications 1 à 7, **caractérisée en ce que** le rapport en poids desdits dérivés de l'urée aux sels de phosphate d'alkyle en C₁₂ à C₂₀ (en particulier aux sels de phosphate de cétyle) se situe dans la gamme allant de 1:0,04 à 1:4, de préférence de 1:0,05 à 1:1, de façon particulièrement préférée de 1:0,075 à 1:0,35.

9. Composition cosmétique selon l'une des revendications 1 à 8, **caractérisée en ce que** les sels de phosphate d'alkyle en C₁₂ à C₂₀ (en particulier les sels de phosphate de cétyle) et les mono- et diacylglycérides en C₁₄ à C₂₀ sont présents dans un rapport en sel de phosphate d'alcyle audit glycéride de 3:1 à 1:1,5, de préférence de 2,5:1 à 1:1.

10. Composition cosmétique selon l'une des revendications 1 à 9, **caractérisée en ce que** des polymères épaississants sont présents dans une quantité de 0 à 0,5% en poids, de manière particulièrement préférée de 0 à 0,4% en poids, de manière tout particulièrement préférée de 0 à 0,1% en poids, le plus préférablement de 0 à 0,05% en poids, à chaque fois par rapport au poids total de la composition.

11. Composition cosmétique selon l'une des revendications 1 à 10, **caractérisée en ce qu'**elle se présente sous la forme d'une émulsion huile-dans-eau.

12. Composition cosmétique selon l'une des revendications 1 à 11, **caractérisée en ce qu'**elle comporte de l'eau dans une quantité de.

13. Composition cosmétique selon l'une des revendications 1 à 12, **caractérisée en ce qu'**elle contient de l'eau dans la phase continue.

14. Composition cosmétique selon l'une des revendications 1 à 13, **caractérisée en ce qu'**elle contient au moins 20% en poids, de préférence au moins 40% en poids, de manière particulièrement préférée d'au moins 50% en poids d'eau, en particulier dans la phase continue.
